# EUROPEAN PATENT APPLICATION

(11) **EP 1 426 447 A1**
(43) Date of publication of application: **09.06.2004**
(21) Application number: 02027272.0
(22) Date of filing: 06.12.2002
(51) Int. Cl.: C12Q 1/14, G01N 33/58, G01N 33/542, C12Q 1/68

(54) **Method for the detection of pathogenic gram positive bacteria selected from the genera Staphylococcus, Enterococcus and Streptococcus**

(71) Applicant: Roche Diagnostics GmbH, 68298 Mannheim (DE); INNOGENETICS N.V., 9052 Ghent (BE)
(72) Inventor: HABERHAUSEN,Gerd, Dr. Dipl.-Biologe, D-82377 Penzberg (DE); EMRICH, Thomas, Dr.,Dipl.-Chemiker, D-82393 Iffeldorf (DE); ROSSAU, Rudi, B-2180 Ekeren (BE); JANNES, Geert, E., B-3010 Leuven (BE); DE VOS, Daniel, B-9260 Schellebelle (BE)

(57) **Abstract**

The present invention is directed to a method for identification of a Gram positive pathogenic bacterium (eg. Staphylococcus, Streptococcus, Enterococcus) comprising (a) an amplification step with at least a first set of amplification primers capable of amplifying a preselected nucleic acid sequence region (such as the 16S-23S rRNA spacer region) from a first predetermined sub-group of pathogenic Gram positive bacteria, (b) a detection step with at least a first hybridization reagent capable of specifically detecting a preselected nucleic acid sequence region from said first predetermined sub-group of pathogenic Gram positive bacteria, said detection step comprising steps (ba) monitoring, whether hybridization has occurred at a preselected temperature, said occurrence of hybridization being indicative for at least the genus of a pathogenic organism present in the sample, and (bb) monitoring temperature dependence of hybridization (using a pair of FRET-probes complementary to adjacent sequences), said temperature dependence being indicative for at least the species of said pathogenic Gram positive bacterium.

SEQUENCE LISTING

<110>  Roche Diagnostics GmbH

<120>  Method for the detection of pathogenic Gram positive Bacteria

<130>  21518 EP

<160>  11

<170>  PatentIn version 3.1

<210>  1
<211>  23
<212>  DNA
<213>  Enterococcus

<400>  1
tactttgttc agttttgaga ggt
23

<210>  2
<211>  24
<212>  DNA
<213>  Enterococcus

<400>  2
gcaattgaac ttattaaaaa actc

24

<210> 3

<211> 21

<212> DNA

<213> Enterococcus

<400> 3
accgagaaca ccgcgttgaa t
21

<210> 4

<211> 29

<212> DNA

<213> Enterococcus

<400> 4
ctggatattg aagtaaaaag aatcaaaac
29

<210> 5

<211> 25

<212> DNA

<213> Control

<400> 5
gatatttgaa gtaaatgtaa gtaat
25

<210> 6

<211> 20

<212>  DNA

<213>  Control

<400>  6
ccagcagaat gccaaccacc
20

<210>  7

<211>  26

<212>  DNA

<213>  Staphylococcus

<400>  7
tgtacattga aaactagata agtaag
26

<210>  8

<211>  22

<212>  DNA

<213>  Staphylococcus

<400>  8
acgcgttatt aatcttgtga gt
22

<210>  9

<211>  23

<212>  DNA

<213>  Staphylococcus

<400> 9
gcttgaattc ataagaaata atc
23

<210> 10

<211> 23

<212> DNA

<213> Staphylococcus

<400> 10
ccgagtgaat aaagagtttt aaa
23

<210> 11

<211> 20

<212> DNA

<213> Control

<400> 11
ccagcagaat gccaaccacc
20

## Description

The invention relates to the technical field of detecting pathogenic microbial organisms. More specifically, the invention relates to the field of detecting an infection caused by a pathogenic Gram positive bacterium in a clinical specimen by means of amplifying and detecting specific nucleic acid sequences from said pathogenic Gram positive bacterium.

### Prior art background

Infection by pathogenic bacteria, particular if causing sepsis, is predominantly occurring and serious in intensive care units (ICU) of hospitals. The bacterium infecting the patient is in most cases unknown and cannot be determined from the symptoms. Each bacterium requires a different therapy using administration of a specific antibiotic. Presently, in routine diagnostics pathogenic bacteria, particularly Gram positive bacteria, are detected using a method including subjecting a sample of blood or other body fluid to culture to grow the bacteria, if present. This culture is maintained at conditions favoring bacterial growth for about three days. During this time, the number of bacteria and thus their nucleic acids is increased. Thereafter, the culture medium is subjected to lysis. The lysis mixture is used as the sample for hybridization reaction. The overall method takes around four days minimum until clarity on any infection of the sample by pathogenic bacteria is reached. Infection by pathogenic bacteria is very serious for the infected person. Within the first day of infection a therapy, preferably by administration of an antibiotic suitable to affect the particular infecting bacterium has to be started. Otherwise, the person is too heavily affected by the infection and may die before clarity on the infection is reached. On the other side, administration of several antibiotics simultaneously to prevent grow of all possible bacteria has to be avoided to not weaken the patient. The present methods thus are not satisfactory for routine ICU diagnostics.

Identification of pathogenic organisms such as pathogenic bacteria or fungi by means of nucleic acid based hybridization using specific hybridization probes has been known in the art already for a long time. For example, EP 0 131 052 discloses methods and probes wherein ribosomal ribonucleic acid (rRNA) sequences of a certain species or a certain group of organisms are detected directly from culture media. Detection of ribosomal target sequences is especially useful due to the fact that these sequences are amplified in-vivo, resulting in high sensitivity of the respective assay.

An improvement of nucleic acid sequence based detection of pathogenic organisms was achieved upon availability of the PCR technology. For the detection of pathogenic fungi such as Candida and Aspergillus, for example, WO 97/07238 discloses a method using generic primers for amplifying all types of fungal ribosomal 18S rDNA sequences and subsequently hybridizing with fungi species specific probes.

As an alternative to the analysis of ribosomal gene sequences, non-coding but transcribed ribosomal spacer DNA sequences like the ITS-1 region located between the 16S and the 23S rRNA genes have been used for detection and identication of several pathogenic organisms (see, for example, EP 0 452 596).

In another context, the groups of Gram positive and Gram negative bacteria have been discriminated by means of a target-dependent amplification comparable to an allele specific amplification approach (Klausegger et al., Journal of Clinical Microbiology 33 (1990) 464-466). On the basis of their 16S r-DNA sequences, the species investigated by Klausegger et al. differ at a given position of the 16S rRNA gene in that all investigated Gram negative bacteria contain a G-residue at a certain nucleotide position whereas all investigated Gram positive bacteria always contain a C-residue at said nucleotide position. Consequently, usage of appropriate primers having either a discriminating complementary 3'-terminal C-residue or a complementary G-residue, respectively, results in DNA amplification of either Gram positive or Gram negative sequence origin.

Further progress was made upon availability of kinetic Real Time PCR. In this type of assay, formation of PCR products is monitored in each cycle of the PCR. The amplification is usually measured in thermocyclers having additional detection means for monitoring fluorescence signals during the amplification reaction. A typical example is Roche Diagnostics LightCycler™ (Cat. No. 2 0110468). In LightCycler™ as well as in other Real Time PCR instruments commercially available so far, amplification products are detected by means of fluorescently labeled hybridization probes which only emit fluorescence signals when they are bound to the target nucleic acid or in certain cases also by means of fluorescent dyes that bind to double-stranded DNA. A defined signal threshold is determined for all reactions to be analyzed and the number of cycles (Cp) required to reach this threshold value is determined for the target nucleic acid as well as for the reference nucleic acids such as a standard or housekeeping gene. The absolute or relative copy numbers of the target molecule can be determined on the basis of the Cp values obtained for the target nucleic acid and the reference nucleic acid (Roche Diagnostics LightCycler™ operator manual(Cat. No. 2 0110468)).

There exist different formats for the detection of amplified DNA:

### a) DNA binding dye format

Since the amount of double stranded amplification product usually exceeds the amount of nucleic acid originally present in the sample to be analyzed, double-stranded DNA specific dyes may be used, which upon excitation with an appropriate wavelength show enhanced fluorescence only if they are bound to double-stranded DNA. Such method is described in EP 0 512 334. Preferably, only those dyes are used, like for example SYBR@ Green I, which do not affect the efficiency of the PCR reaction.

All other formats known in the art require the appropriate design of a fluorescently labeled hybridization probe which only emits fluorescence upon binding to its target nucleic acid.

### b) TaqMan™ probes

A single-stranded hybridization probe is labeled with two components. When the first component, the so-called fluorescer, is excited with light of a suitable wavelength, the absorbed energy is transferred to the second component, the so-called quencher, according to the principle of fluorescence resonance energy transfer. During the annealing step of the PCR reaction, the hybridization probe binds to the target DNA and is degraded by the
5'-3'-exonuclease activity of the polymerase, for example Taq Polymerase, during the elongation phase. As a result the excited fluorescent component and the quencher are spatially separated from one another and thus a fluorescence emission of the first component can be measured (EP B 0 543 942 and US 5,210,015).

### c) Molecular Beacons

These hybridization probes are also labeled with a first component and with a quencher, the labels preferably being located at different ends of an at least partially self-complementary probe. As a result of the secondary structure of the probe, both components are in spatial vicinity in solution. After hybridization to the target nucleic acids both components are separated from one another such that after excitation with light of a suitable wavelength the fluorescence emission of the first component can be measured (US 5,118,801).

### d) FRET hybridization probes

The Fluorescence Resonance Energy Transfer (FRET) hybridization probe test format is especially useful for all kinds of homogenous hybridization assays (Matthews, J. A. and Kricka, L. J., Anal Biochem 169 (1988) 1-25). It is characterized by two single-stranded hybridization probes which are used simultaneously and are complementary to adjacent sites of the same strand of an (amplified) target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured only when both hybridization probes bind to adjacent positions of the target molecule to be detected.

When annealed to the target sequence, the hybridization probes must be located very close to each other, in a head to tail arrangement. Usually, the gap between the labeled 3' end of the first probe and the labeled 5' end or the second probe is as small as possible, i.e. 1-5 bases. This allows for a close vicinity of the FRET donor compound and the FRET acceptor compound, which is typically 10-100 Angstrom. Particulars are well known and disclosed for example in EP 0 070 687.

Alternatively to monitoring the increase in fluorescence of the FRET acceptor component, it is also possible to monitor fluorescence decrease of the FRET donor component as a quantitative measurement of hybridization event.

In particular, the FRET hybridization probe format may be used in real time PCR, in order to detect the amplified target DNA. Among all detection formats known in the art of real time PCR, the FRET-hybridization probe format has been proven to be highly sensitive, exact and reliable (WO 97/46707; WO 97/46712; WO 97/46714). Yet, the design of appropriate FRET hybridization probe sequences may sometimes be limited by the special characteristics of the target nucleic acid sequence to be detected.

As an alternative to the usage of two FRET hybridization probes, it is also possible to use a fluorescent-labeled primer and only one labeled oligonucleotide probe (Bernard, P. S., et al., Anal. Biochem. 255 (1998) 101-7). In this regard, it may be chosen arbitrarily, whether the primer is labeled with the FRET donor or the FRET acceptor compound.

FRET hybridization probes (also called FRET-Hybprobes or FRET probes) can also be used for melting curve analysis (WO 97/46707; WO 97/46712; WO 97/46714). In such an assay, the target nucleic acid is amplified first in a typical PCR reaction with suitable amplification primers The hybridization probes may already be present during the amplification reaction or be added subsequently. After completion of the PCR-reaction, the temperature of the sample is constitutively increased. Fluorescence is detected as long as the hybridization probe is bound to the target DNA. At the melting temperature, the hybridization probe is released from their target, and the fluorescent signal is decreasing immediately down to the background level. This decrease is monitored with an appropriate fluorescence versus temperature-time plot such that the negative of a first derivative function can be calculated. The temperature value corresponding to the obtained maximum of such a function is then taken as the determined melting temperature of said pair of FRET hybridization probes.

Point mutations or polymorphisms within the target nucleic acid result in a less then 100% complementarity between the target nucleic acid and the FRET probes, thus resulting in a decreased melting temperature. This enables for a common detection of a pool of sequence variants by means of FRET-Hybprobe hybridization, whereas subsequently, different members of said pool may become discriminated by means of performing melting curve analysis. Instead of FRET hybrdization probes, Molecular Beacons may alternatively be used for melting curve analysis.

Upon the availability of Real-Time PCR and homogenous Real-Time PCR melting curve analysis, discrimination of certain types of species or strains became possible using either double stranded DNA binding dyes such as SybrGreen™I, or, alternatively, specifically designed hybridization probes hybridizing to different but similar target sequences.

In the first case, melting temperature of the generated double stranded PCR product has to be determined. Yet, this method has only limited applications since differences, because minor sequence variations only result in subtle melting temperature differences, cannot be monitored efficiently. A successful example has been disclosed by Woo et al., Journal of Microbiology 35 (1999) 23-30, who performed amplification of Leptospira biflexa 16S rDNA sequences and subsequent melting curve analysis using SybrGreen™ I as a DNA binding dye in order to discriminate Leptospira biflexa strains from non-specific amplification products originating from the DNA of different Leptospira species.

Alternatively, hybridization probes may be used in such a way that the melting temperature of the probe/target nucleic acid hybrid is being determined. For example, Espy et al., Journal of Clinical Microbiology 33 (2000) 795-799 disclose diagnosis of Herpes simplex infections by means of amplifying a Herpes simplex sequence and subsequent analysis using FRET hybridization probes to discriminate between the HSV Type I and the HSV Type II genotype.

Furthermore, WO 01/48237 suggests in general the connection of amplification and temperature dependent hybridization in order to detect different pathogenic species. Yet, WO 01/48237 does not teach any methods or conditions which enable for an exclusive detection of pathogenic organisms, but to not detect any non pathogenic organism.

Even the presently used methods including in-vitro amplification of specific bacterial species and subsequent detection of said bacterium are not useful for cases where urgent diagnostics is needed, for example in ICU, because for each bacterium an amplification reaction and a detection reaction have to be performed. This requires that a large amount of sample volume, such as blood, has to be drawn from each patient. In ICU large sample volumes from severely infected patients are not available.

Thus, there is a need in the art to provide methods which are specifically applicable for detecting relevant pathogenic organisms of interest. In particular, there is a need in the art for a method which enables for the detection of all relevant pathogenic Gram positive bacteria but at the same time does not detect similar non-pathogenic Gram positive bacteria. Particularly there is a need to determine which pathogenic genus and/or species is present in a sample.

### Brief Description of the invention

The problem disclosed above is resolved by methods and compounds disclosed and claimed in the present application.

Thus, the present invention is directed to a method for identification of a Gram positive pathogenic organism or a subset of organisms being a member of a predetermined group of pathogenic Gram positive bacteria in a clinical sample comprising
a) isolating a clinical specimen containing at least partially purified nucleic acid,
b) subjecting said clinical specimen to at least one amplification and detection comprising
   ba) an amplification step using at least one set of amplification primers capable of amplifying a pre-selected nucleic acid sequence region from a predetermined sub-group of pathogenic Gram positive bacteria to which said Gram positive pathogenic organism belongs,
   bb) a detection step using at least one hybridization reagent capable of detecting said pre-selected nucleic acid sequence region from said predetermined subgroup of pathogenic Gram positive bacteria wherein said detection step bb) comprises steps
   bba) monitoring hybridization at a pre-selected temperature, said hybridization being indicative for the presence in the sample of at least one species contained in said sub-group, and
   bbb) monitoring temperature dependence of hybridization, said temperature dependence being indicative for the presence of at least the species of said pathogenic Gram positive bacterium or said subset of organisms,
c) identifying said organism or said subset of organisms based on the results of the monitoring steps in bb).

The present invention is also directed to compositions suitable for performing the methods disclosed above.

In addition, the present invention is directed to kits suitable for performing the methods disclosed above.

### Detailed description of the invention

The present invention provides methods and compounds especially suitable for the diagnosis of a pathogenic infection of Gram positive bacteria. In this regard, if a patient is suspected to have an infection of a Gram positive bacterium, the present invention provides a means to determine the species of said pathogenic Gram positive organism. Moreover, the new methods according to the invention, especially when combined with Real Time PCR technology such as LightCycler™ system enable for a rapid diagnosis of an infectious agent causing sepsis, which is of outstanding importance in many clinical environments.

Thus, in general, the present invention is directed to a method for identification of a Gram positive pathogenic organism or a subset of organisms being a member of a predetermined group of pathogenic Gram positive bacteria in a clinical sample comprising
a) isolating a clinical specimen containing at least partially purified nucleic acid,
b) subjecting said clinical specimen to at least one amplification and detection comprising
   ba) an amplification step using at least one set of amplification primers capable of amplifying a pre-selected nucleic acid sequence region from a predetermined sub-group of pathogenic Gram positive bacteria to which said Gram positive pathogenic organism belongs,
   bb) a detection step using at least one hybridization reagent capable of detecting said pre-selected nucleic acid sequence region from said predetermined subgroup of pathogenic Gram positive bacteria wherein said detection step bb) comprising steps
   bba) monitoring hybridization at a pre-selected temperature, said hybridization being indicative for the presence in the sample of at least one species contained in said sub-group, and
   bbb) monitoring temperature dependence of hybridization, said temperature dependence being indicative for the presence of at least the species of said pathogenic Gram positive bacterium or said subset of organisms,
c) identifying said organism or a subset of organisms based on the results of the monitoring steps in bb).

In the context of the present invention, the specific terms used above are defined as follows:

The term "identification of a pathogenic Gram positive organism" is used to describe a method to determine whether a particular species or strain or isolate within a species contained in the predetermined group or subgroup of pathogenic Gram positive bacteria is present in the sample. The output or result of the identification is the name of the species or strain or isolate. This will allow subsequently for an appropriate selection of a selective antibiotic therapy.

The term "predetermined group of pathogenic Gram positive bacteria" is used to describe a predefined group of pathogenic Gram positive bacteria consisting of pathogenic Gram positive bacteria which are of interest for a particular task. Preferably, a predetermined group of Gram positive bacteria may be the group of all pathogenic Gram positive bacteria, which are clinically relevant under given clinical circumstances, and the respective genomic sequences of which to become amplified are substantially known in the art.

For example, such a predetermined group may comprise the clinically relevant members of two or more genera. Alternatively, all known clinically relevant members of a certain genus may constitute such a predetermined group. Alternatively, all known clinically relevant members or strains or isolates of a certain species may constitute such a predetermined group. The predetermined group can also contain taxonomic sub-groups of different genera, mixed with strains from other genera or species.

The term "specific" is used to describe the characteristic of a subject (for example in methods, steps or reagents) that the subject is directed to only a particular and defined result. For example, a method for the detection of a particular species is considered to be specific, if only this species, but not other species are detected. Specific hybridization of a probe with a target is a hybridization which only occurs between the probe and the target, but not with other nucleic acids.

In this context, the overall method of the present invention is preferably substantially specific regarding the identification of the organism. Organisms not being a member of the predetermined group or subgroup are preferably not identified, because the steps performed with the reagents are adjusted to not detect organisms not belonging to that group. This does not necessarily mean that each single step of the overall method is specific, while this is possible. For example, if the amplification primers are chose to be not specific for the organism to be identified (in that those primers can be used to amplify nucleic acids of organisms not to be identified), step ba) can be non-specific. Specificity of the overall method can then be achieved by selecting one or more of the parts of the detection step to be specific, for example step bbb). By way of example, the present invention embrasses embodiments, where step bba) results in a positive signal, due to hybridization of the hybridization reagent with a nucleic acid of a member of the predetermined group and in addition to non-target nucleic acids in the specimen or/and amplified in step ba), but in step bbb) monitoring temperature dependence only proves the identity of the species of the member of the predetermined group, independently of the identity of the non-target nucleic acids.

As a main advantage compared to methods known in the art, the present invention allows for the first time a selective identification of the pathogenic members of a bacterial genus or a species, whereas other known, non-pathogenic members of said genus or said species are not identified.

In this context it is important to note that in case a clinical specimen to be analyzed contains a new, previously unknown strain with a new, slightly differing target sequence, the new method according to the invention may occasionally lead to either a false positive or a false negative result. In case an unknown non-pathogenic organism is amplified and detected, the result of step bba) will become false positive. Yet, in this case, the false positive result may become detected during step bbb) of the claimed method, i. e. the monitoring of temperature dependence of hybridization. In this case, comparing the temperature dependency of step bbb) to the temperature dependence of the known pathogenic organisms will show that the organism does not belong to the species of the predetermined group, i.e. the result of step c) will be negative. In case an unknown pathogenic organism is either not amplified or not detected, the result is false negative. Yet according to all studies on clinical isolates performed by the inventors so far, these cases are very rare and, moreover, are not avoided by any other comparable method known in the art.

The term "predetermined sub-group of Gram positive bacteria" is used to describe a part or the whole of the predetermined group of pathogenic bacteria. Preferably, the predetermined subgroup is only a small part of all pathogenic Gram positive bacteria, more preferably the bacteria predominantly occurring in hospitals. The members of the group and the subgroup can be chosen from one genus, but can also be chosen from different genera. In case the predetermined group is represented by all pathogenic and clinically relevant members of a genus, a respective sub-group may contain all respective pathogenic members of a certain species. Similarly, if said predetermined group consists of all clinically relevant members of a certain species, then the sub-group may consist of all members of a specific strain. One very preferred subgroup consists of the species and strains of Staphylococcus aureus, Streptococcus preumoniae, Enterococcus faecium, Enterococcus faecalis, Staphylococcus epidermidis and Staphylococcus haemolyticus (called Group I). Another preferable subgroup consists of Staphylococcus aureus and all Coagulase-negative Staphylococci. Another preferable subgroup consists of Enterococcus faecium and Enterococcus faecalis.

The term "amplification and detection reaction" shall mean any kind of in-vitro method comprising one or more "amplification steps" in order to amplify one or more target sequences from a pool of nucleic acid sequences and one or more "detection steps" in order to unravel the identity of the amplified product.

The term "amplification step" shall mean a reaction or a series of reactions that amplifies, a target sequence - if present in the clinical specimen - by means of using forward and reverse primers for a nucleic acid amplification reaction from a specimen containing DNA. The specificity of said amplification step depends upon the group selected and is governed by the specificity of the primers used. Preferably, amplification is obtained by means of a polymerase chain reaction (PCR) using a thermostable DNA polymerase. In one embodiment, the amplification is specific for bacteria generally; i.e. viruses are not amplified in substantive amounts.

Preferably, the amplification step is specific for the members of the subgroup. In a preferred embodiment, amplification is specific to the genus to which the species to be identified belong.

The term "detection step" shall mean that the amplification product is detected by means of hybridization with an appropriate hybridization reagent, including monitoring temperature dependence of hybridization of said reagent to the target nucleic acid. Amplification and detection do not necessarily be separated and performed serially, but can be performed simultaneously.

The term "pre-selected nucleic acid sequence region" shall mean a distinct target nucleic acid region present in the DNA of all organisms intended to be amplified and detected. Depending on the embodiment, there may exist some sequence variations between the sequences of different organisms. In other words, it is always the same gene or the same homologous sequence of each organism, which is amplified. An example of such a region is the 16S/23S rDNA spacer region, i.e. the region between the sequences coding for the 16S and the 23S rRNA, or a part thereof. More preferably, the preselected nucleic acid sequence region contains at least a part of 20, even more preferred more than 40 contiguous nucleobases from the 16S/23S spacer region of the organisms to be amplified. This region contains evolutionary conserved as well as hypervariable sequences, which allow for a flexible design of both, genus and species specific primers and probe. The region is contained within the region defined by the primer binding sites on the nucleic acid.

The term "pathogenic" means that the bacterium may affect the health status of a human being, if that human being is infected by that bacterium. The gist of the invention is directed to the identification of bacteria causing sepsis. In this case, the predetermined group.

The term "set of amplification primers" shall mean at least two (extendable) oligonucleotides or oligonucleotide derivatives, i. e. at least one (forward) primer binding to a first strand of the target nucleic acid and at least a second (reverse) primer binding to the opposite strand of the target nucleic acid sequence to be amplified. Moreover, the positioning of the primers is designed in such a way that template dependent extension of each primer generates an extension product which itself comprises a primer binding site to which the other primer can hybridize.

In most cases, it is sufficient to use a pair of two amplification primers consisting of one forward primer and one reverse primer. Yet, in some cases there may exist some minor sequence variants in the sequences of the primer binding sites of different sequences of different pathogenic Gram positive bacteria to be identified. Thus it may be impossible to amplify the sequences of all members by just using one forward and one reverse primer. For those cases, a set of amplification primers may consist of 1, 2, or more forward primers and/or 1, 2 or more reverse primers, which are similar and bind to homologous sequences, but differ from each other by one, two, three or several mononucleotide-, dinucleotide- or trinucleotide exchanges, deletions, or additions.

Moreover, it is also within the scope of the invention, if two, three or multiple sets of amplification primers capable of amplifying different pre-selected nucleic acid sequence regions are used. In this case, said different pre-selected nucleic acid sequences may not necessarily be related to each other in sequence. Yet, it is also within the scope of the present invention, if the different pre-selected nucleic acid sequence regions are at least partially or almost completely overlapping.

In general, the design of amplification primers is performed on the basis of available sequence information with regard to the pre-selected target nucleic acid sequence regions of the pathogenic bacteria to be amplified as well as with regard to the homologous sequences of those Gram positive and Gram negative bacteria, which shall not be amplified. More precisely, the set or sets of amplification primers are selected in such a way that there is a maximum sequence complementarity with respect to all target nucleic acid sequences of the selected predetermined group of pathogenic Gram positive bacteria, and, on the other hand, a minimum sequence complementarity with respect to nucleic acid sequences of all other non-selected Gram positive bacteria and Gram negative bacteria, i.e. those not belonging to the predetermined group or not being pathogenic, as well as fungi.

The term "hybridization reagent" is used to describe a reagent capable of hybridizing to products of amplification of step bb) within the preselected nucleic acid sequence region; i.e. on at least one strand of the amplicon(s). The reagent can comprise one or more probes, which preferably are single stranded or are made single stranded prior to hybridization. Preferably the reagent is a single stranded nucleic acid hybridization probe system, comprising usually one or two nucleic acids which are capable of hybridizing to one strand of the double stranded amplified target nucleic acid. Depending on the type of detection format, it is in most cases advantageous if the hybridization reagent is appropriately labelled with a detectable entity, such that by detection of said label, the amplicon/hybridization-reagent hybrid can be detected.

In a preferred embodiment, the hybridization reagent is labelled with a fluorescent entity such that hybridization can be detected in commercially available Real Time PCR instruments. Reagents useful for this are disclosed in the documents mentioned above describing the different formats for the detection of amplified DNA.

The hybridization reagent in a simple case may be an oligonucleotide probe. For example, the Molecular Beacon Format (US 5,118,801) may be applied. Alternatively, it is also possible to use appropriate single labeled oligonucleotides (WO 02/14555). These references are cited to incorporate their content regarding reagents and processes of detection.

More preferably, the hybridization reagent is composed of two adjacently hybridizing oligonucleotides, appropriately labelled such that together they can act according to the FRET-Hybprobe detection format as disclosed above (WO 97/46707; WO 97/46712; WO 97/46714).

Furthermore, in this context, the term "FRET pair" is defined as a pair of fluorescent labels that act together to create a FRET process, i.e. it consists of a FRET donor moiety and a FRET acceptor moiety.

Similar to the design of a set of amplification primers, the design of a hybridization reagent or multiple hybridization reagents is also performed on the basis of all available sequence information with regard to the pre-selected target nucleic acid sequences to be amplified and detected as well as with regard to the homologous sequences of those pathogenic Gram positive bacteria, which shall not be detected. More precisely, the sequences of the hybridization reagent(s) are selected in such a way that there is maximum sequence complementarity with respect to all target nucleic acid sequences of the selected predetermined group of pathogenic Gram positive bacteria, and, on the other hand, a minimum sequence complementarity with respect to all homologous nucleic acid sequences of all other non selected Gram positive bacteria or Gram negative bacteria.

In addition, the design of the hybridization reagent needs to take into account that a discrimination of several target sequence variations is possible on the basis of monitoring temperature dependence of hybridization. The present invention requires different melting temperatures for hybridization of a hybridization reagent to different target sequence variants originating from different pathogenic Gram positive bacteria. Thus, the sequences of a hybridization reagent according to the invention are designed in such a way that the calculated melting temperatures (calculated by methods known in the art) of the different hybridization reagent/target sequence hybrids differ from each other by at least 2°C, preferably by 4°C and most preferably by at least 6°C.

Summarizing, the invention provides a possibility for the design of sub-group, preferably genus specific hybridization reagents, characterized in that they detect all pathogenic members of a certain sub-group genus belonging to a certain predetermined group. Alternatively, species or strain specific hybridization reagents may be designed, which allow for a detection of all pathogenic strains of a certain species belonging to a certain predetermined group.

The term "monitoring temperature dependence of hybridization" shall mean that a characteristic of the reaction mixture is monitored for a certain period of time or in intervals, as required for an intended accuracy of the assay, which changes with and is dependent on the dissociation of a hybrid. Usually, and in conjunction with hybridization assays using labelled probes, the characteristic is a signal influenced by the label on the probe and changed upon hybridization of the probe to an amplicon/target.

In this context the melting temperature (Tm) of a hybrid is defined as the temperature at which the maximum of a first derivative of the hybridization versus temperature signal plot is reached. The Tm is a characteristic of a hybrid depending upon complementarity of the strands.

In the present invention, the annealing temperature of the probe(s) is preferably lower than the melting temperature of the primers used.

A change of characteristics will particularly occur at the or around the melting temperature of each hybrid. The melting temperature is determined by the particular hybridization reagent and the corresponding region at each target to which the reagent binds. In particular, in the method of the present invention, the melting temperature is determined, at which the probe is dissociating from the hybridization complex formed with the target nucleic acid/amplicon. In this context, it is important to note that the melting temperature (Tm) is depending on several factors independent from the actual target nucleic acid sequence itself, such as salt concentration, length and GC-content of the probe. Yet in addition, the melting temperature strongly depends on the number of mismatches, i. e. degree of complementarity between the probe and the target sequence. As a consequence, different melting temperatures are obtained for target sequences found in different strains of a distinct species or different species of a distinct genus. As a consequence, using an appropriate probe design, one type of hybridization reagent (for example one probe) may be used for both the detection of all members of a pre-selected group of organisms and the discrimination of said members by means of monitoring temperature dependence of hybridization. In this case, a probe is used that has a nucleotide sequence complementary to a sequence on the target region of a first Gram positive pathogenic organism, (for example Enterococcus faecium), but slightly different (for example in 1 or 2 nucleotides) from the sequence on the target region on a second Gram positive pathogenic bacterium (for example Enterococcus faecalis.

The term "indicative for at least one species contained in said sub-group" or "...in said the genus" shall mean that if a hybridization signal is occurring in step bba), then it can be concluded for step c) that a pathogenic organism of said sub-group or said certain genus, respectively, may be present in the sample collected from the patient. Depending on the design (for example, the sequence) of the set or sets of amplification primers, and moreover, depending on the design of the hybridization reagent(s), a hybridization signal obtained from the signal of a distinct hybridization reagent may even be indicative for the species of the pathogenic organism to be detected. Preferably, the hybridization signal determined from step bba) is unambiguously indicative for the presence of one of the members of the predetermined group, but does not necessarily directly allow to know which of the members is present.

The term "indicative for at least the species" shall mean that on the basis of the result of monitoring temperature dependence of hybridization as monitored in step bbb) it can unambigously be concluded for step c) which pathogenic species is present in the clinical sample. Due to the design of the set or sets of amplification primers and the design of the hybridization reagent(s), the data obtained from monitoring the temperature dependence of hybridization are indicative for the identity of a species or even a certain strain of the respective pathogenic organism. Even if the specimen may contain non-target sequences similar or identical to the target sequence, they will not be amplified as the primers are not suitable for amplifying the non-target region.

The term ,subset of organisms' is used to describe a predetermined collection of species which are all members of the predetermined group and the predetermined subgroup of pathogenic Gram positive bacteria. Mostly, those organisms belong to one genus. An example of such group is the group of Coagulase negative Staphylococci (CONS) which contains Staphylococcus epidermidis and Staphylococcus haemolyticus or the Streptococcus viridans group which contains Streptococcus mitis, Streptococcus mutans and Streptococcus bovis. The subset preferably contains less than 10, more preferably less than 7 species. The method of the invention in the case of the identification of the presence of an organism present in said subset does not necessarily provide the result which species of said subset is contrained in the clinical sample.

Summarizing, the invention provides a method for the identification of Gram positive pathogenic bacteria, wherein in a first amplification step using one or several appropriate sets of primer pairs sequences of all pathogenic Gram positive bacteria of interest are amplified and subsequently detected by appropriate hybridization reagents. Due to an appropriate primer and probe design, sequences of non-pathogenic Gram positive bacteria or other microbial organisms are either not amplified or not detected, or amplified, but not detected. In case hybridization with a distinct hybridization reagent occurs, it is indicative for at least the sub-group or the genus of the Gram positive infectious agent.

Subsequently, monitoring temperature dependent fluorescence is performed, for example in the form of subjecting the sample to a continuous increase of temperature and determining the temperature at which melting between the target nucleic acid and the hybridization reagent occurs. The monitored melting temperature is then indicative for at least the species or even the sub-species or strain of the respective pathogen that has been present in the original specimen.

Usually, the new method is specifically applicable and useful under circumstances, wherein it has been shown previously that the patient suffers from an infection due to a Gram positive pathogenic bacterium, but the exact identity of said infectious agent is still unknown.

Independently from the definition of the different steps of the method as claimed, it is understood that steps a), b), and c) are preferably performed subsequently one after the other, first step being step a), second step being step b) and third step being step c). Additional steps may be performed before, between and after these steps.

Starting material of the method of the invention is the clinical sample, which is suspected to contain a Gram-positive bacterium. Examples of such clinical samples are whole blood, serum and cerebrospinal fluid. Preferably the starting material is of human origin.

Step a) is always done first and can be performed according to methods known in the art. In this context, a "clinical specimen" is understood as a specimen, preferably a fluid, derived from the clinical sample containing nucleic acids, including nucleic acids of the organism the identification is to be effected.

In order to achieve a sufficient sensitivity for detecting an infection, the method of the invention requires an at least partial purification of the nucleic acid from the original sample. The nucleic acid to be isolated can either RNA or DNA or a mixture thereof. As the most preferably embodiment of the invention uses bacterial DNA for the final identification of the pathogenic organism, it is not required that the clinical specimen contains RNA. Therefore, it is not necessary to partially or even completely isolate RNA from the clinical sample. The isolation of DNA from the clinical sample should be as sufficient and complete as necessary to receive a signal with a positive control.

Particularly, the nucleic acids are separated from proteins, sugars and salts present in the original sample. Any purification method known in the art may be used in the context of the present invention. Preferably, methods are applied which result in an essentially complete purification of the total DNA to be analyzed from other components of the clinical sample. At least, purification should be performed to such an extend that without any further substantial dilution, nucleic acid sequences in aliquots of the specimen can successfully be amplified in an in-vitro amplification, such as PCR. In purification, any compounds substantially inhibiting polymerases are removed from the nucleic acids. Particularly useful methods of nucleic acid isolation are disclosed in WO 96/41811.

The result of step a) usually is a fluid comprising nucleic acids from the original clinical sample and in addition reagents added during the isolation step, like buffers. In step b), the clinical specimen or a part thereof are subjected to one or more amplification reactions. The amplification and detection reactions can comprise one or more steps. Both, amplification and detection reactions are known in the art. The amplification is done using an in-vitro method, preferably PCR (EP 0 201 184). In the following, reference is made to PCR, but it is understood that other in-vitro methods are suitable, too. The result of the amplification reaction is the production of a large number of extension products of said primers, predominantly having a sequence reaching from the 5'-terminal position of one primer to the 5'-terminal position of the other primer. Those nucleic acids produced are usually called "amplicons".

In order to avoid false negative results due to inhibitory residual components which may be present in the clinical specimen and for quantification purposes, it has been proven to be particular advantageous, if an internal control template is added. Usually, said control template comprises a known sequence with primer binding sites complementary to at least one set of amplification primers used for amplification of the target nucleic sequences to be detected. Consequently, said set of amplification primers is also capable of priming amplification of said selected sequence of the control template. Details of using internal standards, particularly for quantification, are disclosed in EP 0 497 784.

It has proven to be advantageous if the method according to the invention is performed using an aliquot of a clinical specimen which has a volume of between 10 and 100 µl. Thus, the method of the present invention can be performed with a sufficient sensitivity, if only a small amount of a clinical sample such as whole blood or serum is available.

Yet, eventually, the results of the inventive method or any other method known in the art may become affected by a high background of human genomic DNA present in a clinical specimen. If this is the case, a pre-amplification step according to WO 01/94634 may be performed, characterized in that selectively non-human DNA sequences are selectively amplified.

For step b) there are both heterogenous and homogenous embodiments possible. In the heterogenous embodiment, the reagents necessary for step ba) are added first and amplification step ba) is performed first. Subsequently, and optionally including supplementation with additional detection reagents, step bb) is performed.

In the homogenous embodiment which is highly preferred over the heterogenous embodiment, reagents for both the amplification and the detection step are added to the specimen prior to the beginning of amplification step ba). In some case, step ba) and step bba) may be performed in parallel, i. e. monitoring of hybridization is performed during amplification, in case of PCR during or after each or at least after several of the performed thermocycles. In this case, preferably said pre-selected temperature of step bba) is usually identical or very similar to the annealing temperature of the PCR thermocycling protocol. The annealing temperature is the temperature at which the hybridization reagent (for example the probe) hybridizes to its target (i.e. the nucleic acid to be amplified, or/and the amplicon formed in earlier extension reactions). In order to achieve sufficient specificity of the hybridization (i.e. to predominantly detect sequences of the target), the annealing temperature is selected such that the probes predominantly anneal/hybridize to the target nucleic acid(s), but not to nucleic acids of organisms not to be identified. Means to influence the selectivity of hybridization of probes to nucleic acids are widely known (length, GC-content and degree of complementarity).

According to the present invention, the amplification step ba) and the detection step bb) are preferably carried out subsequently in a homogenous assay format, characterized in that the one or more hybridization reagents are already present within the reaction mixture during the amplification step. In other words, amplification step ba) and detection step bb) are carried out within the same reaction vessel and without addition of further reagents between the steps.

Yet, alternatively it may be advantageous in at least some cases, if subsequent to the amplification step ba), the reaction mixture is divided into at least two, three, four or several sub-aliquots. Step bb) is then performed with at least the same number of different hybridization reagents, each in a different reaction vessel.

Step bba) is a step wherein the signal of hybridized hybridization reagent is measured and will be done as in conventional homogenous nucleic acid hybridization, particularly as outlined above for the different methods on LightCycler.

Steps bba) and bbb) are preferably done together in such a way that first, the hybridization event itself is monitored at a pre-selected first temperature. Then, the temperature is continuously increased to at least the temperature at which the hybrid containing the hybridization reagent is resolved. In other words, a melting curve analysis for the hybrid is performed.

More precisely, subsequent to the amplification reaction, the PCR product (in the presence of the hybridization reagent) is denatured at a first temperature, for example 90-100°C, and subsequently cooled down to a second temperature, said second temperature being similar or identical to the annealing temperature of the PCR protocol. Then, the temperature is increased at rates between 0,01-10°C/s, preferably 0,1-2°C/s, and most preferably 0,5-1°C/s.

The monitoring step bbb) preferably is performed once a sufficient number of cycles Cp were performed to amplify any target sequence present up to a level to be detectable via temperature dependence. That is usually coinciding with the amount of nucleic acid detectable through probe hybridization, i.e. as soon as there is a clearly positive signal in step bba) measured, step bbb) can be performed. Cp is preferably between 20 and 40.

In case hybridization is monitored during the amplification reaction (which is also called "Real Time"), performance of a melting curve analysis may be omitted, as long as it is not possible to find a hybridization signal at the pre-selected temperature.

Step c) contains interpreting the results which have been obtained during steps bb). This may be done manually by interpretation of the results obtained. Preferably, the results are analyzed using computer proGrams which generate an output that clearly indicates whether and which Gram positive bacterium was present in the sample and thus may have caused the infection.

The interpretation is based on the correlation of the signals obtained in steps bba) and bbb) with values known from positive controls. The use of positive controls is also generally known from the prior art. A positive control is a nucleic acid which is known to be present in the specimen or in an artificially prepared control specimen. For example, the specificity of hybridization of the probe at the preselected temperature is used to determine whether any of the nucleic acids that could have been amplified by the primers in step ba) and which belong to said sub-group of Gram positive pathogenic organisms are present in the specimen. A positive signal (over a negative control) is indicative of the presence of a species belonging to said sub-group (step bba), even if the identity of said species is not determined from said step bba).

The temperature dependence of hybridization is used for identification of the species present in the specimen. By this step, it is determined to which species of said subgroup said organism, the general presence of which is determined from step bba, belongs. For example, its melting temperature is indicative of a nucleic acid of a particular species. Therefore, the presence of a predetermined species in the actual sample can be verified by the occurrence of a change of signal when the melting temperature of the hybrid of the target with the hybridization reagent is reached.

In a first specific embodiment, the predetermined group of pathogenic Gram positive bacteria comprises 2 or more of the following species and strains.

Staphylococcus aureus, Streptococcus preumoniae, Streptococcus pyogenes, Streptococcus agalactiae, Streptococcus viridans group (S. mitis, S. mutans and S. bovis), Enterococcus faecium, Enterococcus faecalis, Staphylococcus epidermidis and Staphylococcus haemolyticus (called Group I).

Preferably, such a method does not substantially detect any other pathogenic and non-pathogenic organism, preferably it does not detect the following species and organisms:

Escherichia coli, Klebsiella pneumoniae, Klebsiella oxytoca, Serratia marcescens, Enterobacter cloacae, Enterobacter aerogenes, Proteus mirabilis, Proteus vulgaris, Pseudomonas aeruginosa, Acinetobacter baumannii, Stenotrophomonas maltophilia, Candida albicans, Candida tropicalis, Candida parapsilosis, Candida krusei, Candida glabrata, Aspergillus fumigatus (called Group II).

Most preferably, such a method detects a group of organisms consisting of all species of Group I, but does not detect any of the organisms belonging to Group II.

In a second specific embodiment, the predetermined group of pathogenic Gram positive bacteria comprises the species and strains of Enterococcus faecium and Enterococcus faecalis, but does not detect any organism of Group II.

In a third specific embodiment, the predetermined group of pathogenic Gram positive bacteria comprises the species and strains of Staphylococcus aureus, Staphylococcus haemolyticus and Staphylococcus epidermidis, but does not detect any organism of Group II. The organisms to be identified are S. aureus (a species) and CoNS (a subset, without differentiating between the species).

This region contains evolutionary conserved as well as hypervariable sequences, which allow for a flexible design of both, genus and species specific primers and probes.

Thus, the present invention is also directed to methods for the detection of pathogenic Gram positive bacteria as disclosed above, wherein at least one set of amplification primers according to SEQ.ID.NO 1 and 2 (Enterococcus) and SEQ.ID.NO. 7 and 8 ( Staphylococcus) are used.

Furthermore, the present invention provides compounds and kits which may be used for identifying pathogenic Gram positive bacteria by means of amplifying and detecting ITS-1 sequences according to methods as disclosed in the present invention.

In one aspect, the present invention provides a composition comprising at least a set of amplification primers, as defined above, preferably having sequences according to SEQ. ID. No: 1 and 2 (Enterococcus) or SEQ.ID.NO: 7 and 8 (Staphylococcus).

In a second aspect, the present invention provides a composition further comprising at least two pairs of FRET hybridization probes, wherein said two pairs are selected from the following group of FRET pairs
SEQ.ID.NO. 3 and 4
SEQ.ID.NO. 5 and 6
SEQ.ID.NO. 9 and 10.

In addition to the ingredients disclosed above, compositions according to the invention may comprise one or several other compounds and reagents selected from the following list:
- Buffer, applicable for a polymerase chain reaction
- Desoxynucleoside triphosphates
- Template dependent DNA polymerase, preferably thermostable,
each separately or in combination.

Further in addition, such a composition may further comprise an at least partially purified nucleic acid, which may e.g. be originated from a clinical specimen and become subjected to any of the methods according to the present invention.

In one aspect, the present invention provides a kit comprising at least one set of amplification primers, said primers having oligonucleotide sequences according to SEQ. ID. No: 1 and 2 (Enterococcus) or SEQ.ID.NO. 7 and 8 (Staphylococcus).

In a second aspect, the present invention provides a kit comprising at least two FRET hybridization probes, wherein said hybridization probes are capable of hybridizing to amplicons created by amplifying at least 20 nucleotides of the 16S/23S rRNA spacer region of any pathogenic organism of any of genera Enterococcus, Staphylococcus and Streptococcus.

In addition to the ingredients disclosed above, kits according to the invention may comprise one, or several other compounds and reagtents selected from the following list:
- Buffer, applicable for a Polymerase Chain Reaction
- Desoxynucleoside triphosphates
- Template dependent DNA polymerase, preferably thermostable

Each of the components disclosed above may be stored in a single storage vesssel. Yet, any combination of components for storage within the same vessel is possible as well.

Furthermore, such a kit may also comprise software tools such as compact discs carrying computer programs for qualitative or quantitative analysis of the data obtained by the claimed method.

The invention differs from prior art bacterial assays in that the known assays only provide information on one particular species or strain (conventional method for the detection of the presence of a particular organism in a sample), the reagents of the invention are suitable for and used for the potential identification of two or more species, while in parallel indicating whether any of them (irrespective which organism) is present.

### List of References

EP 0 131 052
EP 0 452 596
WO 97/46707
WO 97/46712
WO 97/46714
EP0512334
EP 0 543 942
US 5,210,015
EP 0 070 687
EP 0 201 184
EP 0 497 784
WO 01/94634
WO 97/07238
WO 01/48237
WO 02/14555
US 5,118,801
Klausegger et al., Journal of Clinical Microbiology 33 (1990) 464-466 Woo et al., Journal of Microbiology 35 (1999) 23-30
Espy et al., Journal of Clinical Microbiology 33 (2000) 795-799
Matthews, J. A. and Kricka, L. J., Anal Biochem 169 (1988) 1-25
Bernard, P. S., et al., Anal Biochem 255 (1998) 101-7

### Examples

The following examples, references, sequence listing and figures are provided to aid the understanding of the present invention, the true scope of which is set forth in the appended claims. It is understood that modifications can be made in the procedures set forth without departing from the spirit of the invention.

### General:

All oligonucleotides mentioned herein were prepared by chemical synthesis. The reagents for attacking labels can be purchased from Roche Diagnostics GmbH (LightCycler Red 640 NHS Ester Cat.No. 2015161; LightCycler Red 705 Phosphoramidite Cat. No. 2157594; LightCycler Fluorescein (abbreviated 'F' in the following) CPG Cat. No. 3113906). The use of those reagents is described in Biochemica No. 1 (2001), p. 8-13.

All reagents need to be checked for contamination by the organisms to be detected. Only reagents free of those organisms and the nucleic acids originating therefrom can lead to an optimum sensitivity.

### Example 1

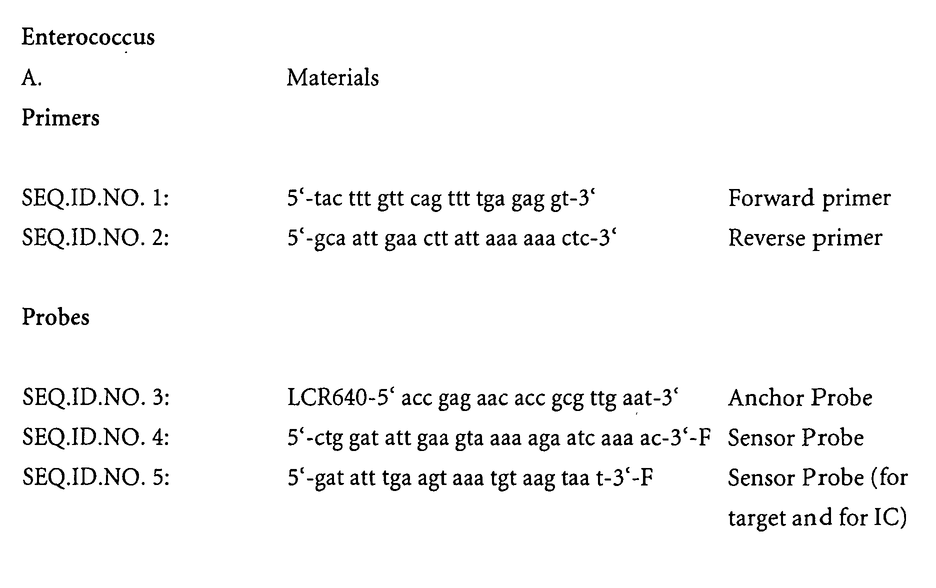

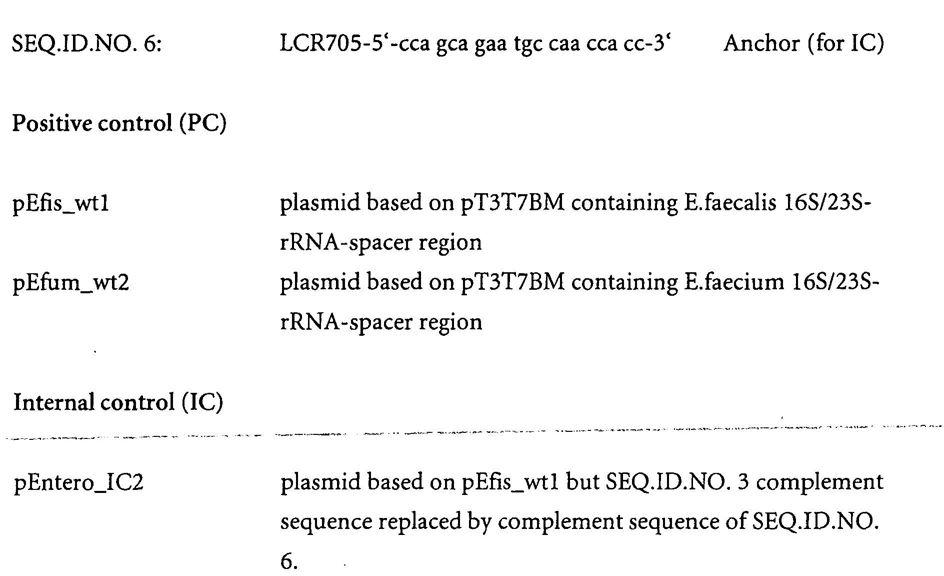

### Hardware / Software

LightCycler™ 1.2 (20µl capillaries) with Software version 3.5 (Roche Diagnostics GmbH, Germany)

| **Reagents** | | |
|---|---|---|
| 1 | FastStart polymerase | |
| 2 | FastStart mastermix containing: | |
| | MgCl₂ | 3.5 mM |
| | Primers | 0.5 µM |
| | Sensor probes | 0.2 µM |
| | Anchor probe | 0.3 µM |
| | Sensor probe (IC) | 0.2 µM |
| 3 | PCR-grade H₂O (purified to be free of cross-contaminating nucleic acids and amplicons of earlier amplifications) | |

FastStart polymerase and FastStart Master are generally used as recommended in the LightCycler-FastStart-DNA Master Hybridization Probes Kit (Roche Diagnostics GmbH Cat.No. 2239272)

### Samples

Samples are either
a. plasmid DNA containing the appropriate binding sequences for primers and probes or
b. preparations of bacterial DNA (e.g. from bacterial culture).

The samples (particularly clinical samples) were subjected to a sample preparation in which the nucleic acids were released and purified from the other components of the sample. This was done using MagNAPure™ LC and MagNA Pure LC DNA isolation kit III (bacteria, fungi)(Roche Diagnostics GmbH, Germany, Cat No. 3 264 785 ). The sample preparation yielded in a specimen containing the nucleic acids in elution buffer.

IC is processed within the sample preparation (e.g. by MagNA Pure) or added to the completed mastermix (see below) dependent on wether it is intended to be an in-process control either for the sample preparation procedure or for LightCycler assay performance in each capillary. The signal from the IC is detected in channel F3 of the LightCycler instrument.

### B. Method

First all components of the FastStart mastermix for the 32 capillaries of the whole rotor were mixed together.
An aliquot of 15µl of the mastermix was then pipetted into each capillary. In the case were the IC was used as a control for the PCR the IC was added to the mastermix. Alternatively, the IC was spiked to the MagNA Pure LC lysis/binding buffer and co-processed with the specimen.
To a first of such capillaries containing the mastermix 5µl of a positive control solution containing a combination of two plasmids with sequence specificity for SEQ.ID.NO. 3 and 4 or SEQ.ID.NO. 5 and 3, respectively, was added.
To a second capillary a "negative control" in the form of 5µl water was added instead of a sample or positive control. The "negative control also contained the IC for in-process control.
After the positiv and negativ controls had been capped the samples (each a volume of 5 µl) were added to other capillaries.

After capping the remaining capillaries a LightCycler-run was performed using the following profile:

| | Cycles | time (sec) | Temp (°C) | Slope(°C) |
|---|---|---|---|---|
| Denaturation | 1 | 600 | 95°C | 20 |
| | | | | |
| Amplification | 45 | 10 | 95 | 20 |
| | | 15 | 50 | 20 |
| | | 10 | 72 | 20 |
| | | | | |
| Melting curve | 1 | 60 | 95 | 20 |
| | | 60 | 40 | 20 |
| | | 0 | 80 | 0.1 |
| | | | | |
| Cooling | 1 | 30 | 40 | 20 |

### C. Results

Analyses were performed in the LightCycler as above. E.faecium and E.faecalis samples showed quantification curves and melting peaks in channel F2 at 54,5°C and 58,5°C, respectively. Most other Enterococci species were not detected. Some rare species (e.g. E.durans, E. hirae, E.mundtii) got a melting peak at lower temperature (< 46°C). Thererfore, the species to be detected (E. faecium and E. faecalis) could be distinguished from each other and all other Enterococcus species present in the sample could be determined by the differring melting temperatures. We found a lower detection limit of at least 10-20 genome equivalent per PCR.
The PC (mixture of pEfis_wt1 and pEfum_wt2 ) showed a quantification curve and a double peak which resulted from a combination of the E. faecium and E. faecalis specific peaks.
The IC allowed the detection in case where there was no specific sample present. It was not detectable in excess of E.faecalis or E.faecium template DNA.

### Example 2

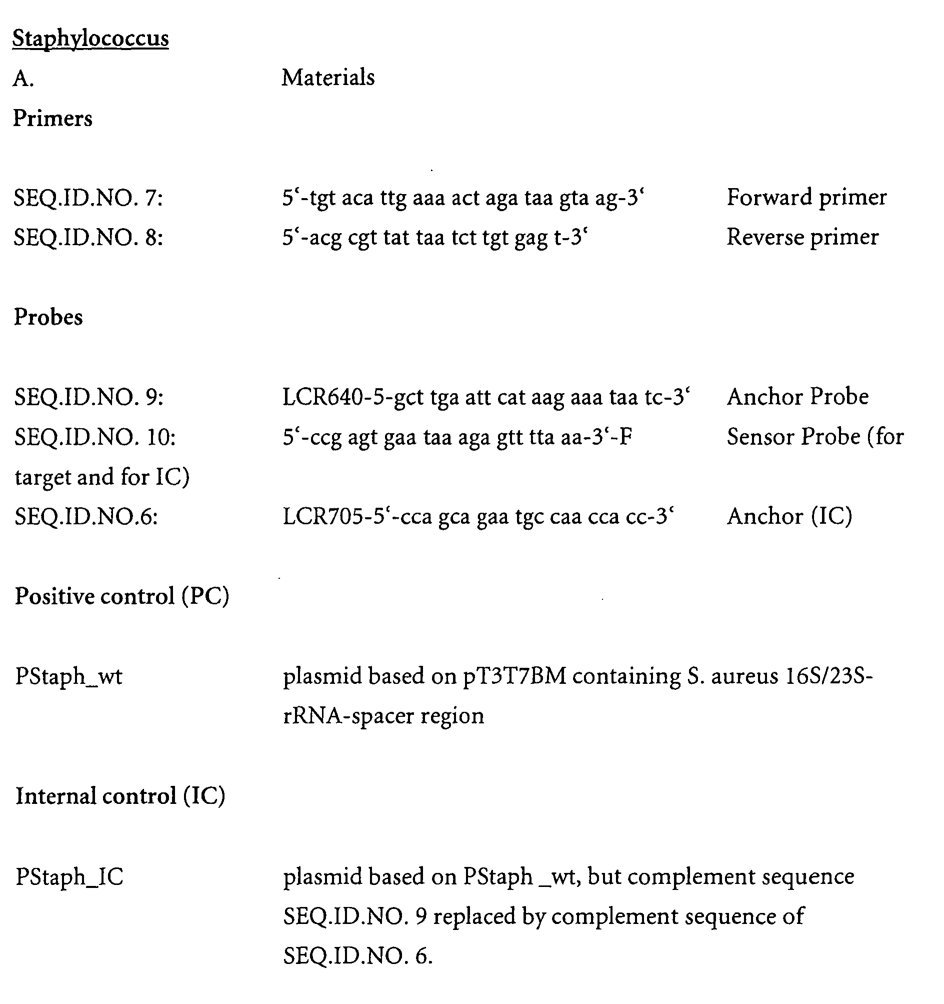

### Hardware / Software

LightCycler™ 1.2 (20µl capillaries) with Software version 3.5 (Roche Diagnostics GmbH, Germany)

| **Reagents** | | |
|---|---|---|
| 1 | FastStart polymerase | |
| 2 | FastStart mastermix containing: | |
| | MgCl₂ | 3.5 mM |
| | Primers | 0.4 µM |
| | Probes | 0.2 µM |
| | Sensor probe (IC) | 0.1 µM |
| 3 | PCR-grade H₂O (purified to be free of cross-contaminating nucleic acids and amplicons of ealier amplifications) | |

FastStart polymerase and FastStart Master are generally used as recommended in the LightCycler-FastStart-DNA Master Hybridization Probes Kit (Roche Diagnostics GmbH Cat.No. 2239272)

### Samples

Samples are either
a. plasmid DNA containing the appropriate binding sequences for primers and probes or
b. preparations of bacterial DNA (e.g. from bacterial culture).

The samples (particularly clinical samples) were subjected to a sample preparation in which the nucleic acids were released and purified from the other components of the sample. This was done using MagNA Pure™ LC and MagNA Pure LC DNA isolation kit III (bacteria, fungi)(Roche Diagnostics GmbH, Germany, Cat No. 3 264 785 ). The sample preparation yielded in a specimen containing the nucleic acids in elution buffer.

IC is processed within the sample preparation (e.g. by MagNA Pure) or added to the completed mastermix (see below) dependent on wether it is intended to be an in-process control either for the sample preparation procedure or for LightCycler assay performance in each capillary. The signal from the IC is detected in channel F3 of the LightCycler instrument.

### B. Method

First all components of the FastStart mastermix for the 32 capillaries of the whole rotor were mixed together.
An aliquot of 15µl of the mastermix was then pipetted into each capillary. In the case were the IC was used as a control for the PCR the IC was added to the mastermix. Alternatively, the IC was spiked to the MagNAPure lysis/binding buffer and co-processed with the specimen.

To a first of such capillaries containing the mastermix 5µl of a positive control solution containing a plasmid with a sequence specificity for SEQ.ID.NO. 9 and 10, respectively, was added.
To a second capillary a "negative control" in the form of 5µl water was added instead of a sample or positive control. The "negative control also contained the IC for in-process control.
After the positiv and negativ controls had been capped the samples (each a volume of 5 µl) were added to other capillaries.
After capping the remaining capillaries a LightCycler-run was performed using the following profile:

| | Cycles | time (sec) | Temp (°C) | Slope(°C) |
|---|---|---|---|---|
| Denaturation | 1 | 600 | 95°C | 20 |
| | | | | |
| Amplification | 45 | 10 | 95 | 20 |
| | | 15 | 50 | 20 |
| | | 10 | 72 | 20 |
| | | | | |
| Melting curve | 1 | 60 | 95 | 20 |
| | | 60 | 40 | 20 |
| | | 0 | 80 | 0.1 |
| | | | | |
| Cooling | 1 | 30 | 40 | 20 |

### C. Results

Analyses were performed in the LightCycler as above. S. aureus and Coagulase-negative Staphylococci (called CoNS) samples showed quantification curves. Melting peaks were observed in channel F2 at about 55°C (S. aureus) and 48-48°C (CoNS), respectively. We found a lower detection limit of at least 10-20 genome equivalent per PCR.
The PC showed a quantification curve resembling the S. aureus quantification and melting peak.
The IC allowed the detection in case where there was no specific sample present. It was not detectable in excess of Staphylococcus template DNA.

## Claims

1. Method for identification of a Gram positive pathogenic organism or a subset of organisms being a member of a predetermined group of pathogenic Gram positive bacteria in a clinical sample comprising
c) isolating a clinical specimen containing at least partially purified nucleic acid,
d) subjecting said clinical specimen to at least one amplification and detection comprising
ba) an amplification step using at least one set of amplification primers capable of amplifying a pre-selected nucleic acid sequence region from a predetermined sub-group of pathogenic Gram positive bacteria to which said Gram positive pathogenic organism belongs,
bb) a detection step using at least one hybridization reagent capable of detecting said pre-selected nucleic acid sequence region from said predetermined sub-group of pathogenic Gram positive bacteria wherein said detection step bb) comprising steps
bba) monitoring hybridization at a pre-selected temperature, said hybridization being indicative for the presence in the sample of at least one species contained in said sub-group, and
bbb) monitoring temperature dependence of hybridization, said temperature dependence being indicative for the presence of at least the species of said pathogenic Gram positive bacterium or said subset of organisms,
c) identifying said organism or said subset of organisms based on the results of the monitoring steps in bb).

2. A method according to claim 1, wherein said sub-group is a genus.

3. A method according to any of claims 1 and 2, wherein the hybridization reagent comprises two probes complementary to adjacent sequences in the target nucleic acid sequence region, one being labelled by a FRET donor, and the other being labelled by a FRET acceptor.

4. A method according to any of claim 1 to 3, wherein said predetermined group of pathogenic Gram positive bacteria comprises the species

5. A method according to any of claims 1 to 4, wherein the predetermined subgroup comprises the species Staphylococcus aureus, Streptococcus preumoniae, Enterococcus faecium and Enterococcus faecalis.

6. A method according to any of claims 1 to 5, wherein the preselected nucleic acid sequence region comprises at least 20 nucleotides of an rRNA spacer region.

7. A method according to any of claims 1 to 6, wherein said amplification and detection is done homogeneously.

8. A method according to any of claims 1 to 7, wherein said species are selected from the genera Staphylococcus, Enterococcus and Streptococcus.

9. A method according to any of claims 1 to 7, wherein said species are selected from one of the genera Staphylococcus.

10. A kit for the identification of a Gram positive pathogenic bacterium selected from the genera Enterococcus, Staphylococcus and Streptococcus containing a set of primers capable of amplifying a sequence of at least 20 nucleotides from the 16S-23S rRNA spacer region of Enterococcus, Staphylococcus and Streptococcus, respectively.
